# EUROPEAN PATENT APPLICATION

(11) **EP 2 690 166 A1**
(43) Date of publication of application: **29.01.2014**
(21) Application number: 12382301.5
(22) Date of filing: 25.07.2012
(51) Int. Cl.: C12G 1/02, C12M 1/04

(54) **Fermentation method and apparatus**

(71) Applicant: Mecánica Logroñesa 71, S.L., 26002 Logrono (ES)
(72) Inventor: Péréz Narcue, Jesús Ángel, E-26002 Logroño (ES); Cantera Miguez, Ricardo, E-26002 Logroño (ES)
(74) Representative: ABG Patentes, S.L.

(57) **Abstract**

The present invention relates to a method for the fermentation of a mixture, particularly in the fermentation of musts and/or grape for wine production, to an apparatus for performing said fermentation, in addition to the maceration, aging and storage of wines, as well as to the use of said apparatus in the maceration, fermentation, aging and storage of grape, must and/or wine, particularly in the fermentation of musts for wine production.

## Description

### Field of the Invention

The present invention relates to a method for the management of grape and/or wine, i.e., maceration of the grape into must and/or the fermentation of must for wine production, to an apparatus for performing said fermentation and/or maceration, in addition to the aging and storage of wine.

### Background of the Invention

The mixture to be fermented in fermentation processes, particularly in wine production, is made up of a liquid part (must) and a solid part, i.e., grape skins and seeds. Large amounts of gases, particularly CO₂, are formed during maceration and fermentation processes, which push the solid parts of the mixture contained in the vat where fermentation takes place towards the upper part, being further compacted, forming a solid layer or cap in the upper part. It is advisable to break said cap to favor the fermentation process and the extraction of substances providing aroma and color to the wine, thereby improving the end product quality.

Mechanical pumping-over processes using mechanical devices and pumps, extracting the wine from one point and distributing it over the upper layer to break the cap and homogenize the fermentation mixture to the greatest extent possible, are currently used in the art. However, said systems have the drawback of breaking the skins and seeds and introducing unwanted components into the end product. Furthermore, pumping-over operations are performed every so often and they are not enough to homogenously mix the must or wine, therefore being stratified. Said stratification results in the production of poor quality wines.

Patent EP 0 979 269 B1 describes a method for the homogenization of the fermentation mixture and the soaking of the cap wherein wasting of the skins is prevented. Said system is based on the accumulation of the CO₂ produced in the tank where fermentation takes place. Said accumulation is achieved by means of placing a diaphragm consisting of a concave surface the function of which is to retain the gas bubbles produced and to subsequently release them when they have become larger. However this method has the limitation of needing a minimum amount of grapes or wine in order to work. The main drawback of this method is that it does not correctly homogenize the wine given that the position of the diaphragm, which is used to internally accumulate the gas produced in fermentation, limits the mixture of the must to that located above it, i.e., it does not allow the must contained in the lower part to circulate to the upper part but simply achieves adequately stirring the part located in the upper part of the cone. Its efficacy can further be reduced if during fermentation the gas volume created is small and does not allow stirring the skins as often as may be desired. Cleaning said fermenter is complex and very uncomfortable, the presence of the diaphragm, bypass, etc., prevents comfortable and efficient cleaning. The high cost for replacing/adapting conventional fermentation tanks by means of incorporating the diaphragm for the accumulation of CO₂ must also be taken into account.

Patent applications WO 2011/161495 A1 and EP 2 058 385 A1 describe a fermentation method comprising fermenting a mixture formed by a solid part and a liquid part in a first tank, extracting the fermentation gas and storing said gas in another tank, then introducing the stored fermentation gas from the first tank into the lower part, such that as the gas bubbles rise, they substantially affect and mix the entire volume of the fermentation liquid. The drawback of said method is that it requires using specially designed tanks to work at high pressures, since it is a closed system. Working in closed systems and at high pressures further leads to the cap hardening, making it difficult to extract CO₂ through the upper part of the fermentation tank. In association with working in closed systems under pressure, there is a risk of explosions caused by a blockage at some point of the system, such as for example the safety valves, because in the medium there are solids, liquid and gas being produced continuously, causing the cap to compact and rise to the upper part of the tank, plugging said safety valves. Another drawback is the need to have a tank for accumulating the fermentation gas for each tank in which fermentation takes place, increasing the space necessary in wineries and accordingly increasing costs, not being able to work continuously, with all this entails in a process where fermentation occurs only once a year. Furthermore, the tanks for producing still wines are not prepared for withstanding dynamic pressures, only hydrostatic pressures, so they cannot be adapted to the proposed system.

The present invention solves the problems of the prior art, mainly improving the efficacy of the homogenization of the fermentation mixture without breaking the skins or the seeds, a high quality end product being obtained, in addition to allowing safer installations as high pressure systems are prevented. The present invention is also economically advantageous because it allows an easy adaptation of existing tanks, is versatile because it allows performing the maceration, fermentation and maturation, and it also optimizes winery space. Furthermore, the present invention allows an accumulation of CO₂ for its later use, thereby covering the needs for said gas in wineries year-round, whereas the systems of the state of the art do not allow such accumulation. Accordingly, the present invention is furthermore advantageous in economic terms (because it gives added value to existing systems without a high cost) and environmental terms (because it largely mitigates the carbon footprint as it uses it as energy which manages and controls the health and quality of the wine, not releasing said CO₂ and also reducing power consumption).

### Summary of the Invention

In a first aspect the invention relates to a method for the fermentation of a fermentable mixture, said mixture comprising a liquid part and optionally a solid part, comprising:
(a)providing a tank (1) suitable for containing the fermentable mixture comprising means (2) for fermentation gas extraction located in the upper part of tank (1), means (3) for fermentation gas compression and means (4) for large gas bubble formation located in the lower part of tank (1);
(b)providing a tank (5) for fermentation gas storage;
(c)introducing the fermentable mixture into tank (1);
(d)extracting the fermentation gas generated during the fermentation and/or maceration of the fermentable mixture through the means (2) for fermentation gas extraction;
(e)compressing the fermentation gas extracted in step (d);
(f)accumulating the fermentation gas compressed in step (e) in tank (5); and
(g) introducing the fermentation gas of tank (5) into tank (1) through the means (4) located in the lower part of tank (1) for large fermentation gas bubble formation of tank (5).

In a second aspect, the invention relates to an apparatus suitable for carrying out the method defined in the first aspect, comprising means for performing steps (a)-(g) defined above.

In a third aspect, the invention relates to an apparatus comprising:
(a)a tank (1) suitable for containing the mixture to be fermented, macerated and/or aged;
(b)means (2) for fermentation gas extraction located in the upper part of tank (1);
(c)means (3) for fermentation gas compression;
(d)a tank (5) for fermentation gas storage;
(e)means (4) for large fermentation gas bubble formation of tank (5) located in the lower part of tank (1); and
(f)control means (11).

In a fourth aspect, the invention relates to a system comprising:
(a)more than one tank (1) suitable for containing the mixture to be fermented, macerated and/or aged, wherein each tank (1) comprises means (2) for fermentation gas extraction located in the upper part of tank (1), means (4) for large fermentation gas bubble formation arranged in the lower part of tank (1) and control means (11);
(b)at least one tank (5) for fermentation gas storage, wherein each tank (5) comprises means (3) for fermentation gas compression;
wherein the number of tanks (1) is greater than the number of tanks (5).

In a fifth aspect, the invention relates to the use of an apparatus as defined in any of the second and third aspects in the fermentation, maceration, aging and/or storage of a mixture comprising a liquid part and optionally a solid part.

In a sixth aspect, the invention relates to a computer program, **characterized in that** it comprises program code means for performing the steps of a method according to the first aspect of the invention when said program runs in a computer.

### Description of the Drawings

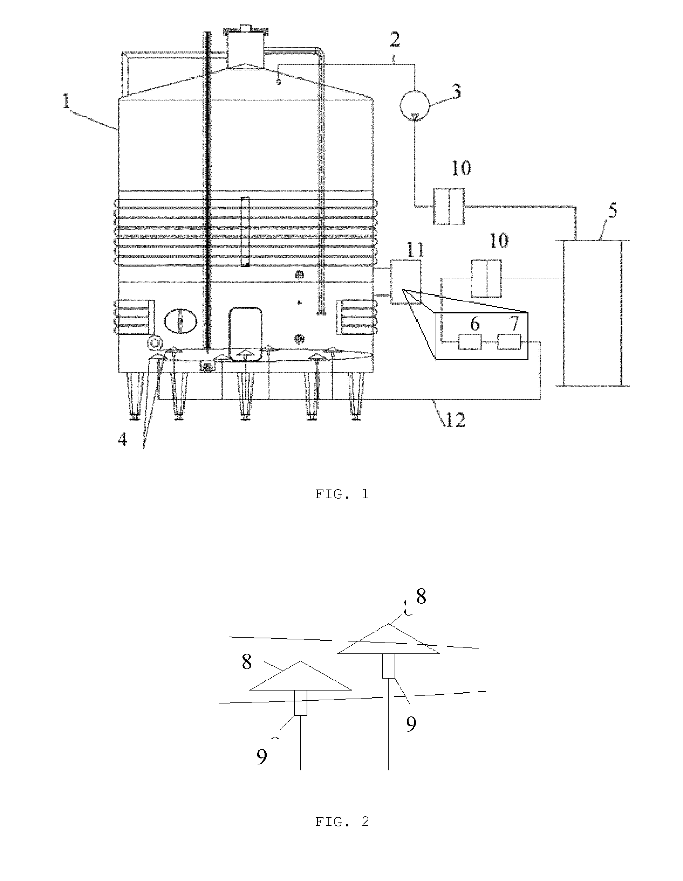

Figure 1 shows a schematic view of a 35000 liter tank (1) comprising means (2) for fermentation gas extraction, means (3) for fermentation gas compression, means (4) for large gas bubble formation, a tank (5) for fermentation gas storage, means (6,7) for fermentation gas flow regulation, two filters (10), a control means (11) and means (12) means for channeling the gas from tank (5) to the means (4) for large gas bubble formation.
Figure 2 shows a schematic view of means (4) for large gas bubble formation comprising a conical element (8) and a non-return valve (9).

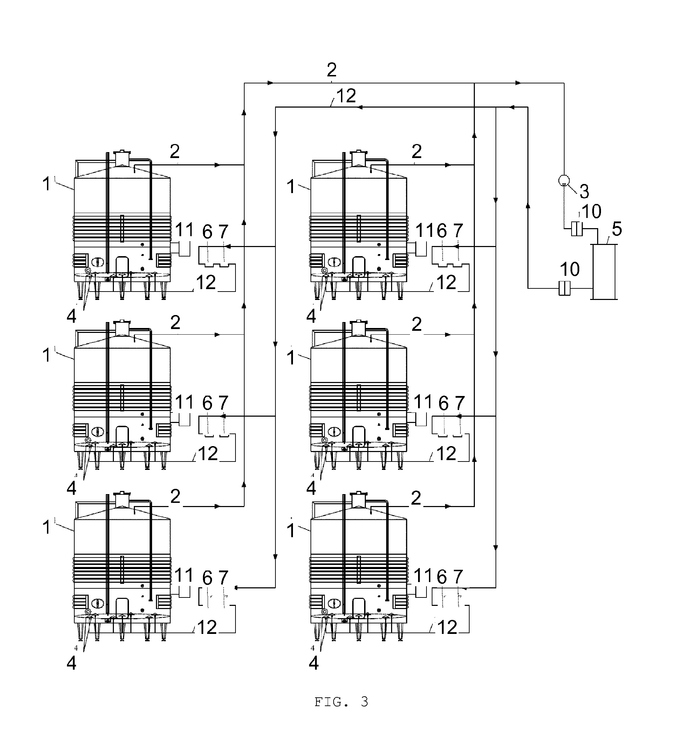

Figure 3 shows a schematic view of a system comprising several tanks (1) comprising means (2) for fermentation gas extraction, common means (3) for fermentation gas compression, means (4) for large gas bubble formation, a tank (5) for fermentation gas storage, means (6,7) for fermentation gas flow regulation, two filters (10) and one control means (11) per tank.

### Detailed Description of the Invention

### Fermentation and/or maceration apparatus

In one aspect, the invention relates to an apparatus comprising:
(a)a tank (1) suitable for containing the mixture to be fermented, macerated and/or aged;
(b)means (2) for fermentation gas extraction located in the upper part of tank (1);
(c)means (3) for fermentation gas compression;
(d)a tank (5) for fermentation gas storage;
(e)means (4) for large fermentation gas bubble formation of tank (5) located in the lower part of tank (1); and
(f)control means (11).

In the context of the present invention, "fermentation" or "fermenting" must be understood as the processing of carbohydrates, generally sugars, such as for example glucose, fructose, sucrose and starch, among others, by microorganisms, particularly yeasts, to obtain as the main end products ethanol and fermentation gases, particularly, carbon dioxide (CO₂). Said fermentation process is used, for example, in producing some alcoholic beverages, such as wine, beer, cider and cava, among others. In a preferred embodiment of the invention, the fermentation gas is CO₂.

In the context of the present invention, "maceration" or "macerating" must be understood as the process taking place immediately before and during fermentation, the exchange of substances between the solid parts of the mixture, particularly skins, seeds and possibly stems of the grape, and the liquid parts of the mixture, particularly the must. In maceration the components contained in the solid fraction are extracted through the must; they primarily provide anthocyanins and tannins, as well as a various aromatic and phenolic substances from the skins, seeds and sometimes the stems, to the fermenting must to give the wine aromas, color and structure.

In the context of the present invention, "aging" must be understood as the controlled process for the aging and maturation of a wine whereby it develops its organoleptic characteristics.

In the context of the present invention, "storage" or "storing" must be understood as the process of saving one or several components in a tank.

In the context of the present invention, "CO₂" must be interpreted as a gas comprising at least 80% in volume of CO₂, preferably at least 85%, preferably at least 90%, preferably at least 95%, more preferably at least 99%. Other gases present in the mixture can be, for example, nitrogen and oxygen.

"Fermentable mixture" or "mixture to be fermented" refers to a mixture which can be fermented, i.e., it comprises carbohydrates, generally sugars, such as for example glucose, fructose, sucrose and starch, among others, and microorganisms, particularly yeasts, the action results in the production of ethanol and fermentation gases, particularly, carbon dioxide (CO₂), as the primary end products. Examples of fermentable mixtures are ground, pressed and/or crushed plant products, such as fruits or grains, such as for example grapes, apples and barley.

"Maceratable mixture" or "mixture to be macerated" refers to a mixture which can be macerated, i.e., it comprises substances, for example aromatic and phenolic substances, which can be extracted. The maceratable mixture comprises a solid part, particularly grape skins, seeds and possibly stems, from which substances are extracted, and a liquid part, particularly the must extracting the substances from the solid part.

In the context of the present invention, the mixture to be macerated, fermented, aged and/or stored comprises a liquid part and optionally a solid part. The fermentable and/or maceratable mixture comes from a plant product that has been ground, pressed and/or crushed, such that the aforementioned solid and liquid parts are obtained. The solid part is present in the maceratable mixture. The solid part is preferably present in the fermentable mixture. The plant product of origin can be any type of fruit or products with sugar, for example, grapes, apples and barley, among others. The plant product is preferably grapes and the end product obtained is preferably wine. In a particular embodiment of the invention, the liquid part of the fermentable and/or maceratable mixture is must and the solid part of the fermentable and/or maceratable mixture is present and comprises skins. In another particular embodiment, the mixture to be aged and/or stored does not comprise a solid part and is preferably wine.

In a particular embodiment of the invention, the tank (1) contains a fermentable mixture comprising a liquid part and a solid part, wherein the liquid part is must and the solid part comprises skins.

In the context of the present invention, the tank (1) (see Figure 1) suitable for containing the mixture to be macerated, fermented, aged and/or stored, preferably macerated and/or fermented, more preferably fermented, can be any conventional tank known in the art, such as commercial stainless steel tanks. The tank can have any shape that is typical in tanks for fermentation and/or maceration, such as cylindrical, rectangular parallelepiped, and cubic, among others. Said tank is preferably cylindrical. The volume of said tank is preferably 100 liters to 200,000 liters, more preferably 5,000 liters to 50,000 liters.

In reference to Figure (1), during the fermentation process, fermentation gases which are displaced to the upper part of the tank (1), from where they are extracted by suitable means (2), are produced.

In the context of the present invention, the means (2) for fermentation gas extraction located in the upper part of tank (1) refer to conventional means known by the person skilled in the art for fluid extraction, specifically for gas extraction, such as one or several suction pipes. The means (2) for fermentation gas extraction are preferably made of stainless steel. Said means (2) are preferably a perimetral suction and discharge installation for said gas. By way of example, the diameter of the suction pipes ranges between 20 mm and 300 mm, and the diameter of the discharge pipes ranges between 10 mm and 100 mm. The means (2) for fermentation gas extraction are in fluid connection with the tank (1) and the means (3) for fermentation gas compression, for example through one or several pipes, as shown in Figure 1.

In the context of the present invention, the means (3) for fermentation gas compression are conventional means known by the person skilled in the art for gas compression which must be understood as an increase in the pressure and reduction of the volume of the gas. The means (3) for fermentation gas compression are preferably mechanical means. Examples of mechanical means (3) for fermentation gas compression are pumps, blowers and compressors absorbing and raising the pressure of the gas inside a pressure accumulator. The means (3) for fermentation gas compression are preferably a compressor. By way of example, a standard compressor which is suitable for food use (for example, coating the gas contact surfaces of the compressor with materials suitable for food use, such as aluminum and Teflon, among others, and without oil losses) can be used for compressing air. The means (3) for fermentation gas compression are in fluid connection with the means (2) for fermentation gas extraction and the tank (5) for fermentation gas storage, for example through one or several pipes, as indicated in Figure 1.

The means (2) and (3) are used when fermentation gases are generated, i.e., in fermentation and aging, as well as during the maceration and storage processes in which fermentation gases are not generated, but the gas (fermentation gas of tank (5)) introduced into tank (1) and used to stir the maceration, aging or storage mixture present in said tank (1), is extracted.

In the context of the present invention, the means (4) for large fermentation gas bubble formation located in the lower part of tank (1) refer to a system which allows injecting fermentation gas from tank (5) into the fermentation mixture in the form of large bubbles. The means (4) for large bubble formation are in fluid connection with tank (5) for fermentation gas storage and tank (1), for example through one or several pipes, as can be seen in Figure 1. The means (4) for large fermentation gas bubble formation of tank (5) are preferably located at the bottom of the inside of tank (1), as shown in Figure 1.

The means (4) are used in any process in which it is necessary to stir the mixture of tank (1), whether it is the maceration, fermentation, aging and/or storage process, preferably the maceration and/or fermentation process, more preferably the fermentation process.

The means (4) for large fermentation gas bubble formation of tank (5) preferably comprise a bubble formation element (8) the lower part of which is in fluid connection with tank (5) through a set of pipes and the upper part of which is facing the upper part of tank (1). Said bubble formation element (8) is concave and allows accumulating fermentation gas in its inner space until filling the volume of said inner space, the accumulated gas then being released in the form of large bubbles. Said means (4) preferably comprise in addition to the bubble formation element (8), a non-return valve (9) located in the base of said element (8), as shown in Figure 2. Said non-return valve (9) must be understood as a valve which completely closes off the passage of fermentation gas in one direction, from tank (1) towards tank (5), whereas it is left clear in the opposite direction, from tank (5) to tank (1). The non-return valves (9) are a safety element between the elements (8) and the external circuit, i.e., the connection with tank (5).

In the context of the present invention, the term "large bubbles" refers to bubbles the volume of which allows stirring the mixture to be macerated, fermented, aged and/or stored, preferably macerated and/or fermented, more preferably fermented, such that the temperature gradients and composition thereof are decreased, and in the particular case that the maceratable and/or fermentable mixture comprises a solid part, the cap which is formed in the upper part of the mixture is also soaked, achieving correct homogeneity. Said bubbles preferably have a size between 5% and 40% of the volume of the tank (1), even more preferably between 10% and 15%.

In the context of the present invention, the tank (5) for fermentation gas storage refers to a conventional tank in the art for the storage of gases under pressure. The tank (5) is in fluid connection on one hand with the compression means (3) and on the other hand with the means (4) for introducing large fermentation gas bubbles, in both cases for example through one or several pipes, as shown in Figure 1. The capacity of the tank (5) ranges between 1,000 liters and 15,000 liters. Said tank (5) is made of stainless steel and works under maximum pressure of 10 bar. A single tank (5) or several tanks can be arranged, depending on the installation and/or needs of the customer, thus allowing the storage of large amounts of the generated fermentation gas, a single tank (5) is preferably used. In a particular embodiment of the invention, the tank (5) has a calibrated safety valve that prevents over-pressure risks.

In one embodiment, the apparatus according to the invention comprises means (6,7) for fermentation gas flow regulation. In a particular embodiment, means (6) are gas pressure regulators and means (7) are electrovalves controlling the injection of the gas into the means (4) for large fermentation gas bubble formation. Said means (6,7) are arranged between tank (5) and tank (1), in the direction of the gas flow, or in the control means (11).

In another embodiment of the invention, the apparatus comprises at least one filter (10) located between the means (2) for fermentation gas extraction and the tank (5), in the direction of the gas flow, more preferably between the means (3) for fermentation gas compression and the tank (5), in the direction of the gas flow, and/or at least one filter (10) located between the tank (5) and the means (4) for large fermentation gas bubble formation, in the direction of the gas flow. An advantage of the present invention is the use of a single tank (5) for storing the extracted fermentation gas. The filter allows removing unwanted components, such as solid particles, and aromas that can be entrained by the fermentation gas to prevent cross-contaminations between several fermenters. Any filter common in the field of the art can be used. The filter is preferably a carbon filter. One or several filters, preferably 1, 2, 3 or 4 filters, more preferably 1 or 2 filters, most preferably 2 filters, can be used. In a particular embodiment, two filters (10) are used, one of them being located between the means (3) for fermentation gas compression and the tank (5), in the direction of the gas flow, and the other one being located between the tank (5) and the means (4) for large fermentation gas bubble formation, in the direction of the gas flow.

The apparatus further comprises control means (11) which allow automating the processes performed by means of said apparatus, preferably control means suitable for carrying out the method of the invention. The times and/or volumes of fermentation gas extracted, compressed and stored, and independently the times and/or volumes of fermentation gas injection, for example, can be programmed. The control means (11) are preferably a control panel, even more preferably a programmable logic controller (PLC) which allows complete management of the process (maceration and/or fermentation, as well as aging and/or storage also), preferably maceration and/or fermentation, more preferably fermentation. In a particular embodiment, the control means (11) comprise a computer program comprising program code means for managing the maceration, fermentation (steps (c)-(g) of the method of the invention), aging and/or storage defined above, preferably maceration and/or fermentation, more preferably fermentation.

In another particular embodiment of the invention, the apparatus further comprises at least one additional tank, said additional tank being in fluid connection (for example, by means of one or several pipes) with the tank (1) or with the means (4) for large bubble formation. Said other gases are, for example, oxygen, external CO₂, argon, nitrogen and/or air. Said additional tank is, for example, a conventional gas storage tank for commercial industrial use.

### Fermentation and/or maceration system

In another aspect, the invention relates to a system comprising:
(a)more than one tank (1) suitable for containing the mixture to be fermented, macerated and/or aged, wherein each tank (1) comprises means (2) for fermentation gas extraction located in the upper part of tank (1), means (4) for large fermentation gas bubble formation arranged in the lower part of tank (1) and control means (11);
(b)at least one tank (5) for fermentation gas storage, wherein each tank (5) comprises means (3) for fermentation gas compression;
wherein the number of tanks (1) is greater than the number of tanks (5).

Said system of fermentation is preferably used in installations having several tanks (1) using only one tank (5) for storing the fermentation gas extracted from said tanks (1) (see Figure 3), and therefore the gas stored in said tank (5) is used to generate large gas bubbles in each of tanks (1) that the person skilled in the art configures for receiving said gas and thus effectively stirring the fermentation, maceration and/or aging mixture, preferably fermentation and/or maceration mixture, more preferably fermentation mixture, in each of said tanks (1). In this particular embodiment, each of tanks (1) comprises means (2) and tank (5) comprises means (3) for fermentation gas compression, the means (2) of each tank (1) being in fluid connection with the means (3) for fermentation gas compression, for example through a set of pipes, and said means (3) being in fluid connection with tank (5), for example through one or several pipes. In turn, each of the tanks (1) comprises means (4) for introducing large fermentation gas bubbles located in the lower part of each of said tanks (1), said means (4) being in fluid connection with tank (5), for example through a set of pipes (12). In turn, each of the tanks (1) comprises control means (11), preferably control means suitable for carrying out the method of the invention. The tanks (1) and (5), as well as the means (2), (3), (4) and (11) have been defined above.

The system preferably comprises between 2 and 30 tanks (1), more preferably between 2 and 20 tanks (1), even more preferably between 2 and 10 tanks (1). The system preferably comprises between 1 and 3 tanks (5), more preferably between 1 and 2 tanks (5), even more preferably a single tank (5).

### Fermentation method

In another aspect, the invention relates to a method for the fermentation of a fermentable mixture, said fermentable mixture comprising a liquid part and optionally a solid part, comprising:
(a)providing a tank (1) suitable for containing the fermentable mixture comprising means (2) for fermentation gas extraction located in the upper part of tank (1), means (3) for fermentation gas compression and means (4) for large gas bubble formation located in the lower part of tank (1);
(b)providing a tank (5) for fermentation gas storage;
(c)introducing the fermentable mixture into tank (1);
(d)extracting the fermentation gas generated during the fermentation of the fermentable mixture through the means (2) for fermentation gas extraction;
(e)compressing the fermentation gas extracted in step (d);
(f)accumulating the fermentation gas compressed in step (e) in tank (5); and
(g)introducing the fermentation gas of tank (5) into tank (1) through the means (4) located in the lower part of tank (1) for large fermentation gas bubble formation of tank (5).

Steps (a) and (b) of the method according to the invention consist of providing the means necessary, i.e., means (2) for fermentation gas extraction located in the upper part of the tank, as defined above, means (3) for fermentation gas compression, as defined above, and means (4) for large fermentation gas bubble formation of tank (5), and necessary tanks, i.e., a tank (1) suitable for containing the fermentable mixture and a tank (5) for fermentation gas storage, as defined above (see Figure 1). The fermentable mixture is then introduced as defined above into tank (1), i.e., step (c) is performed.

Said fermentable mixture comprises microorganisms (particularly yeasts) capable of converting the sugars present in the mixture to be fermented into ethanol. The fermentation process comprises a lag phase, wherein the microorganisms become acclimatized to the conditions of the mixture to be fermented, i.e., concentration of sugars, pH, temperature, etc.; an exponential growth phase, wherein the microorganisms start to multiply exponentially, reaching its maximum population density by means of consuming sugars and their corresponding conversion into ethanol; a stationary phase, wherein the microorganism population has reached a stationary value and fermentation is kept at a constant speed; and a decline phase wherein the scarcity of sugars and/or the concentration of ethanol starts to kill the microorganisms and therefore reduces the fermentation rate. A large amount of fermentation gases, particularly CO₂, is generated during the fermentation process. By way of example, between 40 liters and 60 liters of gas are generated in the must fermentation process for producing wine for every liter of must to produce a wine with an alcohol content between 10 and 15% alcohol. The fermentation gas is produced in the fermentable mixture.

In the method according to the invention, the fermentation gas rises to the surface of the mixture, where the solid part or cap is generally located when the fermentable mixture comprises a solid part, and it accumulates in the fermentable mixture free upper area of tank (1).

In a preferred embodiment of the method according to the invention, the fermentation gas is CO₂. The saturation of the fermentation system with CO₂ as the fermentation gas further has the advantage of protecting the wine against bacterial proliferation and oxidation.

In another preferred embodiment of the method of the invention, the liquid part of the fermentable mixture is must and the solid part of the fermentable mixture is present and comprises skins.

In the method of the present invention, the gas produced in the fermentation is extracted through the means (2) for extraction, it is step (d) of the method. The extracted gas volume is variable and will be determined by the person skilled in the art according to the needs of each particular fermentation method. Between 60% and 80% of the gas produced during fermentation is preferably extracted. Said extraction is performed through the means (2) for extraction defined above. The gas is channeled from the upper part of tank (1) after said extraction and is led, for example through conventional pipes, to the means (3) for fermentation gas compression (see Figure 1).

The next step of the method of the present invention is step (e) or the step of compressing the fermentation gas extracted in the preceding step. Said compression is performed through the means (3) for fermentation gas compression defined above, said means (3) for fermentation gas compression are preferably mechanical means, more preferably a compressor. The compression of a gas refers to a reduction of the volume of a fluid, the fermentation gas in the present invention, with the subsequent increase in the pressure of said gas, i.e., the pressure of the fermentation gas extracted in step (d) is increased. In a preferred embodiment, the pressure of the fermentation gas is increased from atmospheric pressure (this occurs because of the thrust of the gas itself that is being produced) up to 10 bar. After compression the fermentation gas is led from the compression means (3) to the tank (5), for example through one or several conventional pipes (see Figure 1).

Step (f) for accumulating the fermentation gas compressed in the preceding step in tank (5) is then performed. The pressure at which the fermentation gas is stored in tank (5) is comprised between 2 bar and 10 bar. The stored fermentation gas is preferably CO₂.

The fermentation gas stored in tank (5) can be used at any time, i.e., to stir mixtures in maceration processes, in fermentation processes, in subsequent aging and/or storage processes, or even in any other method carried out in the installations where said tank (5) is located, preferably fermentation and/or maceration, more preferably fermentation, requiring the use of said stored fermentation gas, preferably CO₂. This method, for example, allows covering the CO₂ needs of wine-producing installations.

As indicated above, in a preferred embodiment, the means (2) for fermentation gas extraction comprise at least one filter (10) which allows removing possible unwanted components which can be entrained by the fermentation gas, thereby preventing them from being incorporated again in tank (1) or in another tank where it is injected, according to the use of said fermentation gas, preferably CO₂, and thereby preventing cross-contamination between different tanks or equipment where said fermentation gas, preferably CO₂, is used. Any filter common in the field of the art can be used. The filter is preferably a carbon filter. One or several filters, preferably 1, 2, 3 or 4 filters, more preferably 1 or 2 filters, most preferably 2 filters, can be used. In a particular embodiment, two filters (10) are used, one of them being located between the means (3) for fermentation gas compression and the tank (5), in the direction of the gas flow, and the other one being located between the tank (5) and the means (4) for large fermentation gas bubble formation, in the direction of the gas flow.

In the next step of the method according to the invention, step (g), the fermentation gas of tank (5) is introduced into tank (1) through the means (4) located in the lower part of the tank (1) for introducing large fermentation gas bubbles. Said step allows substantially stirring the fermentation mixture, soaking the cap or solid part of said mixture accumulated in the upper part thereof, in the event that the fermentation mixture comprises a solid part, such that it further achieves extracting components from the solid part, circulating the liquid part of the mixture favoring homogenization both with respect to the composition thereof and with respect to the temperature, and they further prevent the compaction of said solid part or hardening of the cap. The process of soaking the solid part of the mixture or cap is very important in wine production because it allows extracting components providing organoleptic characteristics to the wine from said cap. It is important to maintain a homogenous temperature suitable for the microorganisms during fermentation. Heat is produced during fermentation which is generally removed by means of cooling systems such as jackets for example, a temperature gradient being generated in the mixture to be fermented close to the wall of the tank (1) and in the central area thereof. The stirring of the fermentation mixture by means of the large gas bubbles in the method of the present invention completely homogenizes the mass (liquid or liquid and solid) and the temperature of the fermentation mixture, and the optimal performance of the microorganisms (for example yeasts) causing fermentation is further favored.

The large fermentation gas bubbles of tank (5) are formed with the means (4). Upon introducing such bubbles in the lower part of the tank, said bubbles rise up through the fermentation mixture, stirring it until reaching the upper part of the tank again.

In a preferred embodiment of the method of the invention, the means (4) for large fermentation gas bubble formation of tank (5) are located in the lower part of the inside of tank (1), as defined above. Said means (4) preferably comprise a bubble formation element (8), and furthermore a non-return valve (9) preferably located in the base of said element (8), as defined above (Figure 2). The means (4) for large fermentation gas bubble formation of tank (5) are even more preferably located at the bottom of the inside of tank (1).

In a preferred embodiment of the method of the invention, means (6,7) for fermentation gas flow regulation are provided. In a particular embodiment, means (6) are gas pressure regulators and means (7) are electrovalves controlling the injection of the gas into the bubble formation elements (8). Said means (6,7) are arranged between tank (5) and tank (1), in the direction of the gas flow, or in the control means (11).

In another embodiment of the invention, at least one filter (10) is arranged between the means (2) for fermentation gas extraction and the tank (5), more preferably between the means (3) for fermentation gas compression and the tank (5). This particular mode of the method of the invention is especially advantageous when the method is carried out in installations where several tanks (1) and only one tank (5) for storing the fermentation gas extracted from said tanks (1) are used. The filter (10) allows removing unwanted components which can be entrained by the fermentation gas as defined above. Any filter common in the field of the art can be used. The filter is preferably a carbon filter. One or several filters, preferably 1, 2, 3 or 4 filters, more preferably 1 or 2 filters, most preferably 2 filters, can be used. In a particular embodiment, two filters (10) are used, one of them being located between the means (3) for fermentation gas compression and the tank (5), in the direction of the gas flow, and the other one being located between the tank (5) and the means (4) for large fermentation gas bubble formation, in the direction of the gas flow.

In a particular embodiment of the invention, the fermentation method is controlled by means of control means (11), preferably control means suitable for carrying out the method of the invention, which allow automating different steps of the method, as defined above. The times and/or volumes of fermentation gas extracted, compressed and stored in each of steps (d)-(f) can be independently programmed, and the times and/or volumes of fermentation gas introduction in step (e) can also be independently programmed from the preceding steps, for example, can be programmed. In the case of a system comprising several tanks (1), said automation can be programmed either independently for each of the tanks (1) or jointly for all the tanks (1). Control means (11) can be arranged for each of the tanks (1) or there can alternatively be a single control means for the set of tanks (1). There are preferably control means (11) for each of the tanks (1), being able to place each control means next to the tank (1) it controls or alternatively arranging all the control means for each of the tanks (1) in one area, or even integrating said control means (11) for each of the tanks (1) in central control means.

Therefore, another aspect of the present invention relates to a computer program, **characterized in that** it comprises program code means for performing steps (steps (c)-(g)) of the fermentation method according to the invention.

Once fermentation has ended, the fermented liquid is drained out using conventional means in the art, such as for example through an opening at the bottom of the tank by gravity and pumping. Post-fermenting maceration can alternatively be performed before extraction, i.e., keeping the wine and the skins in contact for a larger extraction and concentration thereof, wherein the mixture is stirred by periodically bubbling with the gas of tank (5), as explained above, having the advantage of keeping the mixture in a CO₂ atmosphere which prevents the proliferation of unwanted bacteria.

In one embodiment of the invention, the method is selected from an open or closed method. The method of the invention performed with the tank (1) open to the atmosphere must be understood as "open method". The method of the invention performed with the tank (1) closed to the atmosphere must be understood as "closed method". In a preferred embodiment of the invention, the fermentation process is carried out with the tank (1) open. The advantage of the closed method according to the invention, i.e., when the tank (1) is closed to the atmosphere, compared with other closed methods of the state of the art is that said method is carried out at atmospheric pressure, so it does not require working with specially certified equipment and entails no risks of explosion. The only equipment used in the method of the invention that works at a pressure that is higher than the rest of the equipment is tank (5) for storing the compressed gas. The pressure of tank (5) is a maximum 10 bar, whereas commercial gases are usually stored up to 200 bar. Furthermore, since the system is open, there is no pressure in tank (1), there preferably being a safety relief valve preventing the accumulation of pressure inside said tank (1). The method of the present invention further allows working with the tank (1) open or closed according to the needs at the time, i.e., the tank (1) can initially be closed (closed method) and if there is no need to accumulate more fermentation gas in the tank (5) (for example, because enough fermentation gas, preferably CO₂, has been accumulated or due to the contamination of the fermentation gas generated in tank (1)) tank (1) can be open (open method), just the opposite.

In the method of the invention, steps (d)-(g) are performed continuously until the fermentation process for fermenting the mixture to be fermented of tank (1) has ended. The moment that the fermentation process ends will be determined by the person skilled in the art. In the context of the present invention, it is understood that the fermentation process has ended, for example, by determining the concentration of sugars or ethanol in the mixture present in tank (1), particularly when there is no sugar to be converted into ethanol, and it can be determined by its density of less than 1000 g/liter.

In another particular embodiment of the invention, the method further comprises introducing other gases stored in the additional tank/tanks, said additional tank/tanks being in fluid connection (for example, by means of one or several pipes) with tank (1), or with the means (4) for large fermentation gas bubble formation of tank (5). Said other gases are, for example, oxygen, external CO₂, argon, nitrogen and/or air. Oxygen can be used in wine production (i.e., when the liquid part of the mixture to be fermented is must and the solid part comprises skins), for example, for micro-oxygenation to stabilize the color, decrease astringency, reduce the presence of herbaceous and reductive aromas. Said additional tank is, for example, a conventional gas storage tank.

In another aspect, the invention relates to an apparatus suitable for carrying out the method defined above comprising means for performing steps (a)-(g) of said method.

### Use of the apparatus

In another aspect, the invention relates to the use of an apparatus as defined above in the fermentation, maceration, aging and/or storage, preferably fermentation and/or maceration, more preferably fermentation, of a mixture comprising a liquid part and optionally a solid part, as defined above. The liquid part is preferably must and the solid part is present and comprises skins in the use of the apparatus of the invention in fermentation and/or maceration, preferably fermentation. The liquid part is preferably wine and the solid part is not present in the use of the apparatus of the invention in aging and/or storage. In maceration processes in wine production, the apparatus of the present invention homogenizes the mixture by means of introducing CO₂ accumulated during the fermentation, as explained in relation to the fermentation process, such that the grapes are continuously soaked, a constantly moistened, disaggregated cap allowing maceration of the pulp without breaking the skins or the seeds. In aging and storage processes, the use of the apparatus according to the invention allows stirring the mixture contained in tank (1), generally wine, by means of introducing gas bubbles from tank (5) generated during a fermentation method, while at the same time allowing maintaining a CO₂ atmosphere to prevent the proliferation of unwanted bacteria.

## Claims

1. A method for the fermentation of a fermentable mixture, said fermentable mixture comprising a liquid part and optionally a solid part, comprising:
(a)providing a tank (1) suitable for containing the fermentable mixture comprising means (2) for fermentation gas extraction located in the upper part of tank (1), means (3) for fermentation gas compression and means (4) for large fermentation gas bubble formation located in the lower part of tank (1);
(b)providing a tank (5) for fermentation gas storage;
(c)introducing the fermentable mixture into tank (1);
(d)extracting the fermentation gas generated during the fermentation of the fermentable mixture through the means (2) for fermentation gas extraction;
(e)compressing the fermentation gas extracted in step (d);
(f)accumulating the fermentation gas compressed in step (e) in tank (5); and
(g) introducing the fermentation gas of tank (5) into tank (1) through the means (4) located in the lower part of tank (1) for large fermentation gas bubble formation of tank (5).

2. The method according to claim 1, wherein the means (4) for large fermentation gas bubble formation are arranged in the lower part of the inside of tank (1), preferably at the bottom of the inside of tank (1).

3. The method according to any of the preceding claims, wherein means (6,7) for fermentation gas flow regulation arranged between tank (5) and tank (1), in the direction of the gas flow, or in the control means (11) are further provided in step (a).

4. The method according to any of the preceding claims, wherein the means (3) for fermentation gas compression are mechanical means.

5. The method according to any of the preceding claims, wherein the fermentation gas is CO₂.

6. The method according to any of the preceding claims, wherein the liquid part of the fermentable mixture is must and the solid part of the fermentable mixture comprises skins.

7. The method according to any of the preceding claims, wherein the method is selected from the open method and the closed method.

8. An apparatus suitable for carrying out the method defined in any of claims 1 to 7 comprising means for performing steps (a)-(g) defined in claim 1.

9. Apparatus comprising:
(a) a tank (1) suitable for containing the mixture to be fermented, macerated, and/or aged;
(b)means (2) for fermentation gas extraction located in the upper part of tank (1);
(c)means (3) for fermentation gas compression;
(d)a tank (5) for fermentation gas storage;
(e)means (4) for large fermentation gas bubble formation of tank (5) arranged in the lower part of tank (1); and
(f)control means (11).

10. Apparatus according to claim 9, wherein the means (4) for large fermentation gas bubble formation of tank (5) are arranged in the lower part of the inside of tank (1), preferably at the bottom of tank (1).

11. Apparatus according to any of claims 9 to 10, further comprising means (6,7) for fermentation gas flow regulation arranged between tank (5) and tank (1), in the direction of the gas flow, or in the control means (11).

12. Apparatus according to any of claims 9 to 11, wherein the means (3) for fermentation gas compression are mechanical means.

13. A system comprising:
(a)more than one tank (1) suitable for containing the mixture to be fermented, macerated and/or aged, wherein each tank (1) comprises means (2) for fermentation gas extraction located in the upper part of tank (1), means (4) for large fermentation gas bubble formation arranged in the lower part of the tank (1) and control means (11);
(b)at least one tank (5) for fermentation gas storage, wherein each tank (5) comprises means (3) for fermentation gas compression;
wherein the number of tanks (1) is greater than the number of tanks (5).

14. Use of an apparatus as defined in any of claims 8 to 12 or of the system as defined in claim 13, in the fermentation, maceration, aging and/or storage of a mixture comprising a liquid part and optionally a solid part.

15. A computer program, **characterized in that** it comprises program code means for performing the steps of a method according to any of claims 1 to 7 when said program runs in a computer.
